# EUROPEAN PATENT SPECIFICATION

(11) **EP 2 928 445 B1**
(45) Date of publication and mention of the grant of the patent: **31.01.2018**
(21) Application number: 13801559.9
(22) Date of filing: 04.12.2013
(51) Int. Cl.: A61K 8/35, A61K 8/37, A61K 8/40, A61Q 3/02, A61K 8/55, A61K 8/73, A61K 8/87, A61K 8/81

(54) **NAIL MAKEUP METHOD WITH PHOTOCROSSLINKABLE VARNISH COMPOSITIONS**
PHOTOVERNETZBARE NAGELZUSAMMENSETZUNG UND VERFAHREN ZUR HERSTELLUNG
PROCEDE DE MAQUILLAGE DES ONGLES AVEC UNE COMPOSITION PHOTORETICULABLE

(30) Priority: 05.12.2012 FR 1261678; 18.01.2013 US 201361754061 P
(43) Date of publication of application: 14.10.2015
(73) Proprietor: L'Oréal, 75008 Paris (FR)
(72) Inventor: KERGOSIEN, Guillaume, F-92370 Chaville (FR); RIACHI, Carl, F-75013 Paris (FR); LE PAPE, Marina, 92120 Montrouge (FR)
(74) Representative: Lavoix
(86) International application number: PCT/EP2013/075543
(87) International publication number: WO 2014/086867

(56) References cited:
- WO-A2-2011/011304
- US-A1- 2011 060 065
- US-A1- 2011 082 228

## Description

The present invention relates to a makeup method of a nail and/or false nail by applying photocrosslinkable varnish compositions.

Nail varnish compositions may be used as a base for the varnish (or *basecoat*), as a nail makeup product, or as a finishing composition (or *top-coat*) to be applied on the nail makeup product, or as a cosmetic nail care product. These compositions may be applied onto natural nails as well as onto false nails.

In the field of nail varnishes, liquid cosmetic compositions are known, which are used by first applying a coat onto the nail and then subjecting said coat to the action of light radiation, inducing *in situ* polymerization and/or crosslinking reactions within said coat, resulting in generally crosslinked polymeric networks. Such photocrosslinkable compositions, commonly referred to as "UV gels" and generally based on (meth)acrylate monomer type crosslinkable compounds, are suitable for obtaining a satisfactory stability of the coat applied on the nail, and are described for example in CA 1 306 954, US 5 456 905, US 7 375 144 and FR 2 823 105.

Some products substantially free from (meth)acrylate monomers are also on the market. However, these products pose performance problems particularly in respect of makeup quality and varnish stability over time.

The aim of the present invention is to provide a novel method using photocrosslinkable compositions which do not have the drawbacks of the aforementioned alternative compositions.

In particular, the aim of the present invention is to provide a novel makeup method using photocrosslinkable compositions, preferably substantially free from (meth)acrylate monomers, which have a satisfactory stability and a high gloss in relation to the photocrosslinkable compositions currently available, notably those substantially free from (meth)acrylate monomers.

The aim of the present invention is to provide a novel makeup method using photocrosslinkable compositions, preferably substantially free from (meth)acrylate monomers, which are easy to use.

Another aim of the invention is to obtain photocrosslinkable compositions suitable for providing coats having the following properties: stability over time (with a mild etching or without any etching of the nail or false nail before applying the composition), easy makeup removal, high cosmeticity, outstanding makeup result (homogeneous deposition, easy to apply, comfortable to wear) and/or high gloss.

The present invention relates to a makeup and/or care method of a nail and/or false nail, comprising the following steps:
a) applying, onto a nail or false nail, a photocrosslinkable cosmetic composition C1 preferably comprising less than 10% by weight of (meth)acrylate monomer in relation to said composition C1, whereby a coat consisting of at least one layer of said composition C1 is deposited;
   the composition C1 comprising in a physiologically acceptable medium:
   - at least one photocrosslinkable (meth)acrylate compound,
   - at least one film-forming polymer P1 in a proportion greater than or equal to 10% by weight in relation to the dry extract weight of the composition C1,
   - at least one volatile solvent S in a proportion greater than or equal to 30% in relation to the total weight of the composition C1, and
   - at least one photoinitiator,
b) exposing the coated nail or false nail obtained following step a) to UV or visible light radiation, whereby photocrosslinking is carried out to obtain a crosslinked layer C'1;
c) applying, onto the nail or false nail coated with the crosslinked layer C'1, obtained following step b), a photocrosslinkable cosmetic composition C2 preferably comprising less than 10% by weight of (meth)acrylate monomer in relation to said composition C2, whereby a coat consisting of at least one layer of said composition C2 is deposited;
   the composition C2 comprising in a physiologically acceptable medium:
   - at least one photocrosslinkable urethane di(meth)acrylate compound at a content by weight greater than or equal to 65% in relation to the total dry extract weight of C2, and
   - at least one volatile solvent S';
d) exposing the coated nail or false nail obtained following step c) to UV or visible light radiation, whereby photocrosslinking is carried out to obtain a crosslinked layer C'2.

According to one embodiment, the compositions C1 and C2 according to the invention are photocrosslinkable compositions comprising a reduced quantity of (meth)acrylate monomer. In this way, they comprise preferably less than 5%, or less than 2%, or more preferentially less than 1%, by weight of (meth)acrylate monomer in relation to the total weight of said composition.

According to one advantageous embodiment, the compositions C1 and C2 are substantially free from (meth)acrylate monomer. Advantageously, the compositions C1 and C2 are completely free from (meth)acrylate monomer.

The term "(meth)acrylate monomer" refers to a compound comprising a single (meth)acrylate function according to the formula H₂C=C(R)-C(O)-O-, where R = H or CH₃.

### Physiologically acceptable medium

The compositions C1 and C2 according to the invention comprise a physiologically acceptable medium.

The term "physiologically acceptable medium" refers to a medium that is particularly suitable for the application of a composition of the invention onto keratin matter.

The physiologically acceptable medium is generally suitable for the nature of the support to which the composition should be applied, and also for the way in which the composition is to be packaged.

### Photocrosslinkable (meth)acrylate compound

The composition C1 comprises at least one photocrosslinkable (meth)acrylate compound as defined above. It may thus comprise a single photocrosslinkable (meth)acrylate compound or a mixture of a plurality of photocrosslinkable (meth)acrylate compounds.

Within the scope of the present invention, the term "photocrosslinkable compound" refers to an organic compound suitable for crosslinking under the action of a light ray, resulting in a crosslinked polymer network.

The term "(meth)acrylate compound" refers to any compound comprising at least one (meth)acrylate function according to the formula H₂C=C(R)-C(O)-O-, where R = H or CH₃.

The "urethane" function is also referred to as a "carbamate" function.

According to one embodiment, the photocrosslinkable (meth)acrylate compound is a photocrosslinkable compound comprising at least two (meth)acrylate functions and at least one carboxylic acid function.

These compounds are defined by the presence of at least one carboxylic acid function, i.e. one -COOH function, and at least two (meth)acrylate functions, i.e. H₂C=C(R)-C(O)-O- functions, where R = H or CH₃.

According to one embodiment, these photocrosslinkable compounds comprise at least two carboxylic acid functions and at least two (meth)acrylate, preferably methacrylate, functions.

According to one embodiment, these photocrosslinkable compounds comprise at least two carboxylic acid functions and four (meth)acrylate functions, preferably four methacrylate functions.

According to one embodiment, the photocrosslinkable (meth)acrylate compound of C1 is of formula (I): 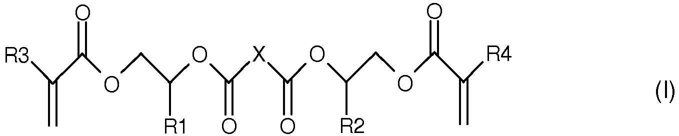 wherein:
- R1 and R2, identical or different, represent a hydrogen atom, or a methyl group, or a group of formula (II): 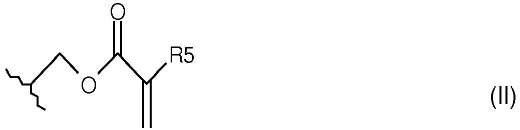 where R5 represents a hydrogen atom or a methyl group;
- R3 and R4, identical or different, represent a hydrogen atom or a methyl group;
- X represents a radical of any of the following formulae (III), (IV), (V), (VI), (VII), (VIII), (IX), or (X): 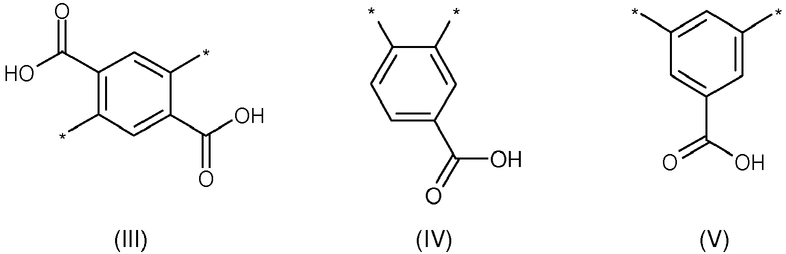 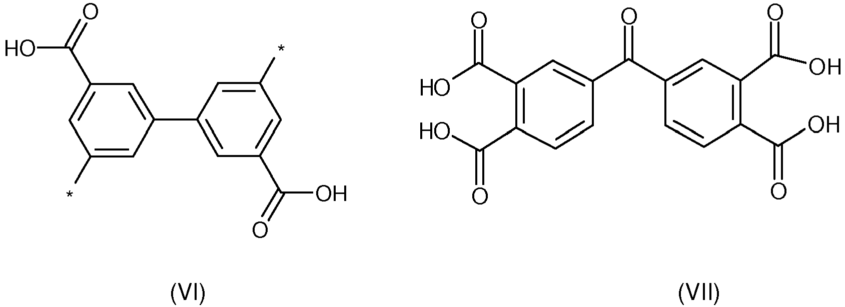 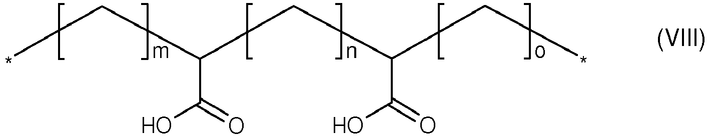 where m, n, and o, identical or different, represent an integer between 0 and 10; 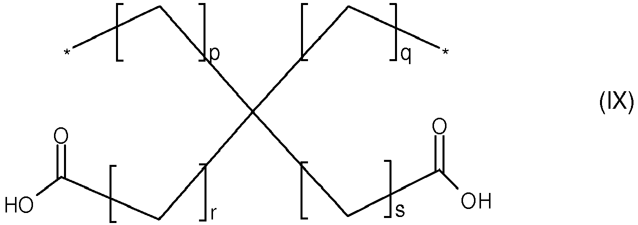 where p, q, r and s, identical or different, represent an integer between 0 and 10; 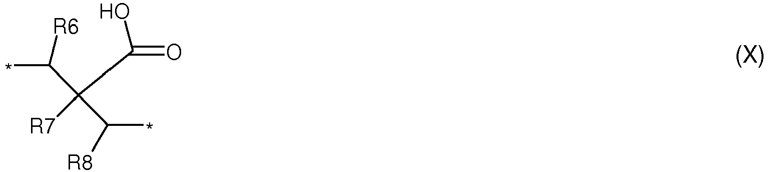 where R6, R7 and R8, identical or different, represent a hydrogen atom or a hydroxyl group.

In the above formulae, the * symbols represents the binding sites of the -X-radical.

According to one embodiment, the photocrosslinkable (meth)acrylate compound of C1 complies with formula (I) as defined above, wherein the radical X is an aromatic radical, particularly an arylene radical, and preferably a phenylene radical.

Preferably, the photocrosslinkable (meth)acrylate compound of C1 is of formula (I-1): 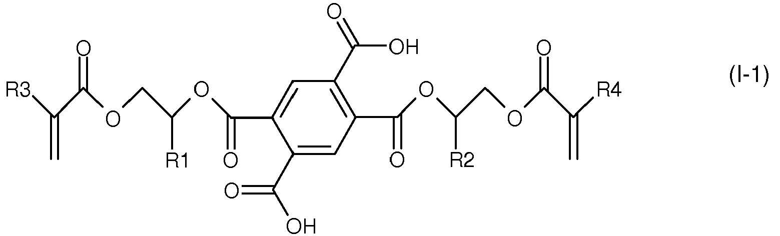 where R1, R2, R3 and R4 are as defined above in formula (I).

According to one embodiment, the photocrosslinkable (meth)acrylate compound of C1 is of formula (I) or (I-1) as defined above, wherein R3 and R4 are methyl groups.

According to one embodiment, the photocrosslinkable (meth)acrylate compound of C1 is of formula (I) or (I-1) as defined above, wherein R1 and R2, identical or different, represent a group according to the following formula (II-1):

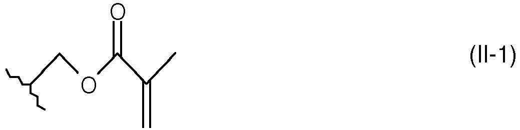

Preferentially, the photocrosslinkable (meth)acrylate compound of C1 is of formula (I-2):

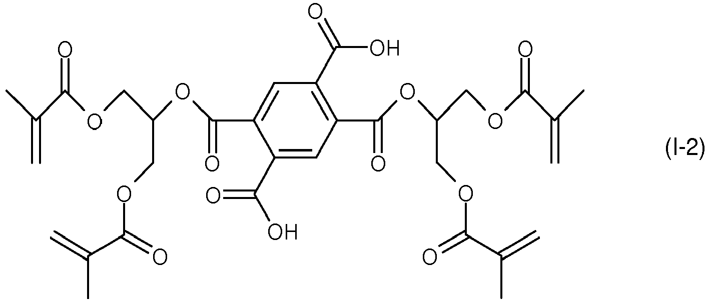

According to one embodiment, the photocrosslinkable (meth)acrylate compound of C1 is a urethane (meth)acrylate compound.

The term "urethane (meth)acrylate compound" refers to any compound comprising at least one urethane function -O-C(O)-NH-, and at least one (meth)acrylate function according to the formula H₂C=C(R)-C(O)-O-, where R = H or CH₃.

As examples of urethane (meth)acrylate compounds, mention may be made of urethane poly(meth)acrylate compounds, particularly urethane di(meth)acrylate compounds, and more particularly urethane dimethacrylate compounds.

According to the present invention, the term "poly(meth)acrylate compound" refers to a (meth)acrylate compound comprising a plurality of (meth)acrylate functions.

In this way, the term "poly(meth)acrylate compound" may refer to a compound comprising at least two methacrylate functions, or at least two acrylate functions, or at least one methacrylate function and at least one acrylate function.

Advantageously, the mean number of (meth)acrylate functions borne by the photocrosslinkable urethane (meth)acrylate compound intended to form, after crosslinking, a crosslinked polymeric network, is greater than 1. Indeed, a polymerizable system consisting of molecules each bearing a single (meth)acrylate function forms, after reacting all of said functions, a linear or branched, and not crosslinked, chain macromolecular system. Only the presence of a certain fraction of molecules bearing at least two (meth)acrylate functions and thus acting as a crosslinking agent is suitable for obtaining a crosslinked polymeric system.

In the implementation of the present invention, the mean number of (meth)acrylate functions per molecule of urethane (meth)acrylate compound is preferably greater than or equal to 2, advantageously ranging from 2 to 6, preferably from 2 to 4, and more preferentially equal to 2.

Preferentially, this urethane (meth)acrylate compound is a urethane dimethacrylate compound. The term "urethane dimethacrylate compound" refers to any compound comprising at least one urethane function -O-C(O)-NH-, and two methacrylate functions according to the formula H₂C=C(CH₃)-C(O)-O-.

According to one embodiment, the composition C1 may comprise a mixture of photocrosslinkable (meth)acrylate compounds. According to one embodiment, the composition C1 may comprise a urethane (meth)acrylate compound as defined above and a photocrosslinkable (meth)acrylate compound comprising at least two (meth)acrylate functions and at least one carboxylic acid function, particularly being of formula (I) or (I-1) as defined above.

According to one embodiment, the composition C1 has a content of photocrosslinkable (meth)acrylate compound(s) as defined above ranging from 1% to 20%, and preferably from 5% to 10%, by weight in relation to the total weight of C1.

### Film-forming polymer P1

The composition C1 comprises at least one film-forming polymer, or at least two film-forming polymers.

In this way, the compositions C1 may comprise a single polymer P1 or a mixture of a plurality of polymers P1.

Preferably, the compositions according to the invention comprise a mixture of polymers P1. According to this embodiment, they comprise at least two film-forming polymers.

According to one advantageous embodiment, the compositions according to the invention comprise two polymers P1 and P'1.

The film-forming polymer content is at least 10% by weight in relation to the dry extract weight of the composition. Preferably, this content is greater than or equal to 30%, or 40%, and preferably greater than or equal to 50%, by weight in relation to the dry extract weight of C1.

The term "film-forming polymer" refers to, according to the invention, a polymer suitable for forming alone (i.e. in the absence of an auxiliary film-forming agent or an external stimulus for example such as UV), a film suitable for being isolated, particularly a continuous adherent film, on a substrate, particularly on nails.

This film-forming polymer may be chosen from the group consisting of radical or polycondensate type synthetic polymers, polymers of natural origin, and mixtures thereof.

A film-forming polymer suitable for the invention may be chosen from polysaccharide derivatives, such as cellulose or guar gum derivatives. One preferential polysaccharide derivative suitable for the invention may be nitrocellulose or a polysaccharide ester or alkylether.

The term "polysaccharide ester or alkylether" refers to a polysaccharide consisting of repeat units comprising at least two identical or different rings and having a degree of substitution per saccharide unit between 1.9 and 3, preferably between 2.2 and 2.9, and more particularly between 2.4 and 2.8. The term substitution refers to the functionalization of hydroxyl groups into ester and/or alkylether functions, and/or the functionalization of carboxyl groups into ester functions.

In other words, it may consist of a polysaccharide, partially or totally substituted with ester and/or alkylether groups. Preferably, the hydroxyl groups may be substituted with C₂-C₄ ester and/or alkylether functions.

Particular mention may be made of cellulose esters (such as cellulose acetobutyrates or cellulose acetopropionates), cellulose alkylethers (such as ethylcelluloses), and ethylguars.

A film-forming polymer suitable for the invention may be chosen from synthetic polymers such as polyurethanes, acrylic polymers, vinyl polymers, polyvinylbutyrals, alkyd resins and ketone/aldehyde resins, resins from aldehyde condensation products, such as aryl sulfonamide formaldehyde resins such as toluene sulfonamide formaldehyde resin, aryl-sulfonamide epoxy resins or ethyl tosylamide resins.

In particular, it may consist of(meth)acrylate homopolymers and copolymers.

A film-forming polymer suitable for the invention may also be chosen from polymers of natural origin, such as plant resins such as dammars, elemi, copals, benzoin; gums such as shellac, sandarac and mastic.

As a film-forming polymer, the toluene sulfonamide formaldehyde resins "Ketjentflex MS80" from AKZO or "Santolite MHP", "Santolite MS 80" from FACONNIER or "RESIMPOL 80" from PAN AMERICANA, the alkyd resin "BECKOSOL ODE 230-70-E" from DAINIPPON, the acrylic resin "ACRYLOID B66" from ROHM & HAAS, the polyurethane resin "TRIXENE PR 4127" from BAXENDEN, the acetophenone/formaldehyde resin marketed under the reference Synthetic Resin SK by Degussa may notably be used.

According to one preferred particular embodiment, the film-forming polymer is chosen from the group consisting of polysaccharides and polysaccharide derivatives, preferably from nitrocellulose and polysaccharide ethers and esters, particularly C₂-C₄, and more preferentially from cellulose acetobutyrates, cellulose acetopropionates, ethylcelluloses, ethylguars, and mixtures thereof.

According to one advantageous embodiment, the film-forming polymer is chosen from the group consisting of nitrocellulose, cellulose acetopropionate, cellulose acetobutyrate, and (meth)acrylate homopolymers and copolymers, and mixtures thereof.

According to one embodiment, the compositions C1 comprise at least nitrocellulose, if applicable in association with a further film-forming polymer as defined above.

Preferentially, in the compositions C1, the polymer P1 is a mixture of nitrocellulose and a (meth)acrylate copolymer. Preferably, according to this embodiment, the ratio between the weight of nitrocellulose and the weight of (meth)acrylate copolymer is less than or equal to 1, and preferentially between 0.5 and 1.

### Volatile solvent S of C1

The composition C1 comprises at least one volatile solvent S. It may thus comprise a single solvent or a mixture of a plurality of volatile solvents.

The mass solvent S content is preferably between 40% and 80%, preferably between 50% and 70%.

The term "volatile solvent" refers to a solvent capable of evaporating on contact with keratin matter, in less than one hour, at ambient temperature and at atmospheric pressure.

The volatile solvent(s) according to the invention are liquid solvents at ambient temperature, having a vapor pressure different to zero, at ambient temperature and atmospheric pressure, particularly ranging from 0.13 Pa to 40,000 Pa (from 10⁻³ to 300 mm Hg), particularly ranging from 1.3 Pa to 13,000 Pa (from 0.01 to 100 mm Hg), and more specifically ranging from 1.3 Pa to 1300 Pa (from 0.01 to 10 mm Hg).

Preferably, the solvents S are chosen from polar solvents.

The term "polar" solvent, according to the present invention, refers to a solvent, or an oil, wherein the solubility parameter calculated over the melting point δₐ thereof is different to 0 (J/cm³)^{½}.

The definition and calculation of HANSEN three-dimensional solubility parameters are described in the article by C. M. HANSEN: "The three dimensional solubility parameters" J. Paint Technol. 39, 105 (1967).

According to the Hansen space:
- δ_{D} characterizes the LONDON dispersion forces derived from the formation of dipoles induced during molecular shocks;
- δₚ characterizes the DEBYE interaction forces between permanent dipoles and the KEESOM interaction forces between induced dipoles and permanent dipoles;
- δₕ characterizes the specific interaction forces (such as hydrogen, acid/base, donor/acceptor bonds, etc.); and
- δₐ is determined by the equation: δₐ= (δₚ²+ δₕ²)^{½}.

The parameters δₚ, δₕ, δ_{D} et δₐ are expressed in (J/cm³)^{½}.

In particular, the term "polar" solvent refers to a solvent wherein the chemical structure is essentially formed from, or consists of, carbon and hydrogen atoms, and comprising at least one highly electronegative heteroatom such as an oxygen, nitrogen, silicon or phosphorus atom.

Preferably, this polar volatile solvent is chosen from the group consisting of C3-C6 esters and ketones and mixtures thereof.

As an example of a polar volatile solvent, mention may be made of acetone, methylethylketone, methylisobutylketone, cyclohexanone and alkyl acetates wherein the alkyl group comprises from 2 to 5 carbon atoms, such as methyl acetate, ethyl acetate, propyl acetate, n-propyl acetate, isopropyl acetate, n-butyl acetate, isobutyl acetate and tert-butyl acetate.

Preferably, the polar volatile solvent is C3-C5, and more preferentially chosen from the group consisting of ethyl acetate, n-propyl acetate, isopropyl acetate and mixtures thereof.

According to one preferred embodiment, the solvent S is ethyl acetate.

### Photoinitiator

The composition C1 comprises at least one photoinitiator.

It may comprise a single photoinitiator or a mixture of a plurality of photoinitiators.

According to one embodiment, the composition C1 comprises two photoinitiators.

The photoinitiators suitable for use according to the present invention are known in the art and are described, for example in "Les photoinitiateurs dans la reticulation des revetements", G. Li Bassi, Double Liaison - Chimie des Peintures, No. 361, November 1985, p.34-41; "Applications industrielles de la polymerisation photoinduite", Henri Strub, L'Actualité Chimique, February 2000, p.5-13; and "Photopolymères: considerations théoriques et reaction de prise", Marc, J.M. Abadie, Double Liaison - Chimie des Peintures, No. 435-436, 1992, p.28-34.

These photoinitiators include:
- α-hydroxyketones, marketed for example under the names DAROCUR® 1173 and 4265, IRGACURE® 184, 2959, and 500 by BASF, and ADDITOL® CPK by CYTEC,
- α-aminoketones, marketed for example under the names IRGACURE® 907 and 369 by BASF,
- aromatic ketones marketed for example under the name ESACURE® TZT by LAMBERTI. Mention may also be made of thioxanthones marketed for example under the name ESACURE® ITX by LAMBERTI, and quinones. These aromatic ketones generally require the presence of a hydrogen donor compound such as tertiary amines and particularly alkanolamines. Mention may particularly be made of the tertiary amine ESACURE® EDB marketed by LAMBERTI.
- α-dicarbonyl derivatives of which the most common is benzyl dimethyl ketal marketed under the name IRGACURE® 651 by BASF. Further commercial products are marketed by LAMBERTI under the name ESACURE® KB1, and
- acylphosphine oxides, such as for example bis-acylphosphine oxides (BAPO) marketed for example under the names IRGACURE® 819, 1700, and 1800, DAROCUR® 4265, LUCIRIN® TPO, and LUCIRIN® TPO-L by BASF.

Preferably, the photoinitiator is chosen from the group consisting of α-hydroxyketones, α-aminoketones, aromatic ketones preferably associated with a hydrogen donor compound, aromatic α-diketones and acylphosphine oxides, and mixtures thereof.

A mixture of photoinitiators absorbing light radiation at various wavelengths is preferably used in C1. The absorption spectrum of the photocrosslinkable composition can thus be adapted to the emission spectrum of the light sources used.

Preferably, the composition C1 comprises a mixture of two different photoinitiators, such as for example a mixture of an α-hydroxyketone and an acylphosphine oxide.

As a photoinitiator mixture, mention may be made of a mixture of IRGACURE® 184 (BASF) and LUCIRIN® TPO-L (BASF).

A particular group of photoinitiators suitable for use in the composition C1 according to the present invention is that of copolymerizable photoinitiators. It consists of molecules comprising both a photoinitiator group capable of photoinduced radical splitting and at least one double ethylene bond. The photoinitiators in this group offer the advantage, in relation to the conventional photoinitiators listed above, of being suitable for being incorporated, via the double bond, into the macromolecular system. This possibility reduces the content of free residual photoinitiators not having undergone photoinduced radical splitting and thus enhances the safety of the layer of varnish.

As examples of such copolymerizable photoinitiators, mention may be made of benzophenone acrylate derivatives marketed by CYTEC under the names EBECRYL® P36, EBECRYL® P37.

In one preferred embodiment of the invention, polymer photoinitiators or photoinitiators bound onto a high molar mass molecule are used. The choice of such a high mass photoinitiator offers the same advantage as selecting only polymeric copolymerizable compounds, i.e. enhanced safety of the photocrosslinkable cosmetic compositions due to the absence of very reactive molecules liable to diffuse to neighboring biological substrates. The mean molar mass by weight of the photoinitiator is preferably at least equal to 500 g/mol.

For example, mention may be made of an α-hydroxyketone oligomer corresponding to the following formula: 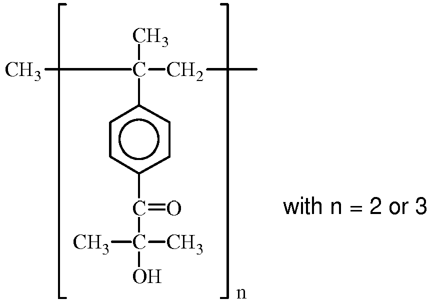 and which is marketed under the name ESACURE® KIP 150 by LAMBERTI.

The polymer on which the photoinitiator group is bound may optionally comprise one or a plurality of double ethylene bonds for optionally incorporating, into the macromolecular network, photoinitiator molecules not having undergone photoinduced splitting.

As examples of such high molar mass photoinitiators bearing double ethylene bonds, mention may be made of those corresponding to the following formulae: 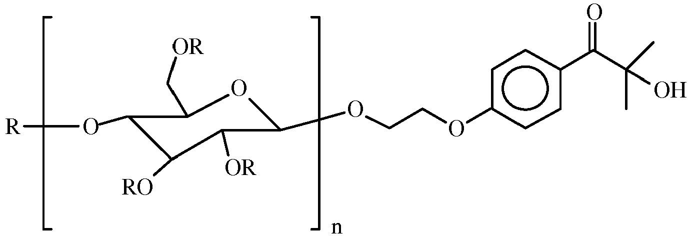 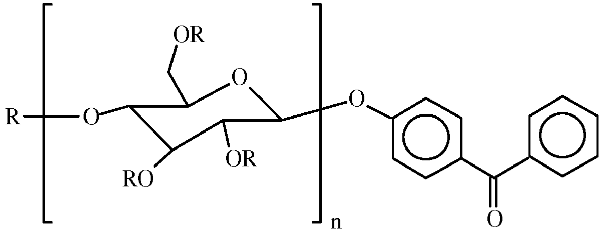 with n = 1 to 20 ; R = H or

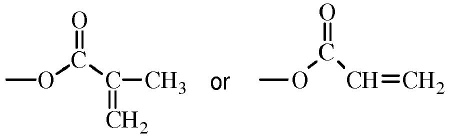

These structures are described in the following articles: S. Knaus, Pure Appl. Chem., A33(7), 869 (1996); S. Knaus, J. Polym. Sci, Part A = Polym. Chem., 33, 929 (1995); and R. Liska, Rad'Tech Europe 97, Lyon, F, 1997, Conference Proceedings.

The photoinitiator content is dependent on a large number of factors such as the reactivity of the various constituents of the mixture, the presence of pigments or dyes, the crosslinking density sought, the intensity of the light source or the exposure time.

In order to obtain satisfactory mechanical properties, the photoinitiator(s) is (or are) preferably present in a total content greater than or equal to 0.1% by weight in relation to the total weight of the composition C1, preferably ranging from 1 to 5% by weight in relation to the total weight of the composition C1.

### Photocrosslinkable urethane (meth)acrylate compound

The composition C2 comprises at least one photocrosslinkable urethane di(meth)acrylate compound. It may thus comprise a single photocrosslinkable urethane di(meth)acrylate compound or a mixture of photocrosslinkable urethane di(meth)acrylate compounds.

Preferably, the composition C2 comprises two photocrosslinkable urethane di(meth)acrylate compounds.

The urethane di(meth)acrylate compounds of C2 are as defined above in relation to the composition C1.

The composition C2 comprises at least 65% by weight of photocrosslinkable urethane di(meth)acrylate compounds in relation to the total dry extract weight of C2.

Preferably, the composition C2 comprises at least 70%, preferably at least 75%, and preferentially at least 80%, by weight of photocrosslinkable urethane di(meth)acrylate compounds in relation to the total dry extract weight of C2.

### Volatile solvent S'

The composition C2 comprises at least one volatile solvent S'. It may thus comprise a single solvent or a mixture of a plurality of volatile solvents.

The mass solvent S' content is preferably between 10% and 30%, preferably between 15% and 25%.

Preferably, S' chosen from polar solvents.

Preferably, this polar volatile solvent is chosen from the group consisting of C3-C6 esters and ketones and mixtures thereof.

As a preferred solvent S', mention may be made of the solvents cited above for the solvent S.

According to one preferred embodiment, the solvent S' is butyl acetate.

### Film-forming polymer P2

The composition C2 may also comprise at least one film-forming polymer P2.

Preferably, the composition C2 comprises a single film-forming polymer P2.

The film-forming polymer content is preferably between 0.5% and 10%, preferably between 1% and 5%, by weight in relation to the total weight of C2.

The film-forming polymer P2 of C2 is as defined above for the polymer P1 of C1.

According to one advantageous embodiment, the film-forming polymer P2 is chosen from the group consisting of nitrocellulose, cellulose acetopropionate, cellulose acetobutyrate, and (meth)acrylate homopolymers and copolymers, and mixtures thereof.

Preferentially, in the compositions C2, the film-forming polymer P2 is a (meth)acrylate copolymer.

### Adjuvants

The compositions C1 and C2 may further contain adjuvants, or additives, particularly chosen from pigments and dyes, plasticizers, coalescing agents, preservatives, waxes, thickeners, perfumes, UV filters, cosmetic active substances for nail care, spreading agents, anti-foaming agents and dispersing agents.

Obviously, those skilled in the art will take care to choose these optional adjuvants or additives such that the advantageous properties of the composition according to the invention are not, or are practically not, altered by the envisaged addition.

If the composition comprises pigments and/or dyes, it is particularly advisable to adapt the absorption spectrum of the pigments and/or dyes used to that of the photoinitiators, or conversely the absorption spectrum of the photoinitiators to that of the pigments and/or dyes used, so as to prevent both types of compounds from absorbing light at the same wavelengths. Indeed, the absorption of light by the pigments and/or dyes would render the photoinitiators present beyond a specific depth of the coat almost completely ineffective.

The compositions C1 and C2 according to the invention may further comprise one or a plurality of plasticizers.

According to one embodiment, these compositions comprise less than 15% by weight of plasticizer in relation to the total weight of said composition.

Preferably, the mass plasticizer content ranges from 0% to 15%, preferentially from 1% to 10%, and more preferentially from 5% to 10%.

As examples of plasticizers, mention may particularly be made of standard plasticizers such as glycols and derivatives thereof, such as diethylene glycol ethylether, diethylene glycol methylether, diethylene glycol butylether or diethylene glycol hexylether, ethylene glycol ethylether, ethylene glycol methylether, ethylene glycol butylether, ethylene glycol hexylether, glycol esters, propylene glycol derivatives and particularly propylene glycol phenylether, propylene glycol diacetate, dipropylene glycol butylether, tripropylene glycol butylether, propylene glycol methylether, dipropylene glycol ethylether, tripropylene glycol methylether and diethylene glycol methylether, propylene glycol butylether, acid esters particularly carboxylic acid esters, such as citrates, particularly triethyl citrate, tributyl citrate, triethyl acetylcitrate, tributyl acetylcitrate, triethyl-2 hexyl acetylcitrate; phthalates, particularly dimethoxyethyl phthalate; phosphates, particularly tricresyl phosphate, tributyl phosphate, triphenyl phosphate, tributoxyethyl phosphate; tartrates, particularly dibutyl tartrate; adipates; carbonates; sebacates; benzyl benzoate, butyl acetyl ricinoleate, glyceryl acetyl ricinoleate, butyl glycolate, camphor, glycerol triacetate, N-ethyl-o,p-toluenesulfonamide, oxyethylenated derivatives such as oxyethylenated oils, particularly plant oils, such as castor oil, silicone oils, hydrocarbon oils, and mixtures thereof.

Preferably, the composition C1 is transparent.

As used herein, the term transparent refers to that the composition has a HAZEBYK index of less than 5 as measured with a KYKHAZEGLOSS type gloss meter.

According to one advantageous embodiment, the composition C2 comprises at least one coloring agent. Preferably, the composition C2 is a colored composition.

According to one embodiment, the composition C2 further comprises a coloring agent chosen from the group consisting of soluble dyes, pigments, nacres and glitter.

The coloring agent(s) may be present in a total content greater than or equal to 0.1% by weight in relation to the total weight of the layer, ranging preferably from 0.1 to 5%, advantageously from 0.2 to 1% by weight in relation to the total weight of C2.

The term "soluble dyes" should be understood to refer to organic, inorganic or organometallic compounds, soluble in the composition according to the invention and intended to color said composition.

The dyes are, for example, Sudan Red, DC Red 17, DC Green 6, β-carotene, soybean oil, Sudan brown, DC Yellow 11, DC Violet 2, DC Orange 5 and Quinoline Yellow.

The term "pigments" should be understood to refer to inorganic or organic, white or colored particles of any shape, insoluble in the composition according to the invention and intended to color said composition.

The term "nacres" should be understood to refer to iridescent particles of any shape, particularly produced by some mollusks in their shell or by synthetic means.

The pigments may be white or colored, inorganic and/or organic. Of the inorganic pigments, mention may be made of titanium dioxide, optionally surface-treated, zirconium or cerium oxides, along with zinc, iron (black, yellow or red) or chromium oxides, manganese violet, ultramarine blue, chromium hydrate and iron blue, metallic powders such as aluminum powder, copper powder.

Of the organic pigments, mention may be made of carbon black, D & C type pigments, and lacquers based on cochineal carmine, barium, strontium, calcium, aluminum.

Mention may also be made of effect pigments such as particles comprising a natural or synthetic organic or inorganic substrate, for example glass, acrylic resins, polyester, polyurethane, polyethylene terephthalate, ceramics, aluminas and optionally coated with metallic substances such as aluminum, gold, copper, bronze, or with metal oxides such as titanium dioxide, iron oxide, chromium oxide, inorganic or organic pigments and mixtures thereof.

The pearlescent pigments may be chosen from white pearlescent pigments such as mica coated with titanium, or bismuth oxychloride, colored pearlescent pigments such as titanium mica coated with iron oxides, titanium mica coated with iron blue and chromium oxide in particular, titanium mica coated with an organic pigments of the aforementioned type and pearlescent pigments based on bismuth oxychloride.

Pigments with goniochromatic properties may be used, particularly liquid crystal or multilayer pigments.

Optical brighteners or fibers optionally coated with optical brighteners may also be used.

The compositions C1 and C2 may further comprise one or a plurality of fillers, particularly at a content ranging from 0.01% to 50% by weight, in relation to the total weight of the composition, preferably ranging from 0.01% to 30% by weight.

The term "fillers" should be understood to refer to inorganic or synthetic colorless or white particles of any shape, insoluble in the medium of the composition regardless of the temperature at which the composition is manufactured. These fillers may particularly be used to modify the rheology or texture of the composition.

The fillers may be mineral or organic particles of any shape, in sheet, spherical or oblong form, regardless of the crystallographic shape (for example sheet, cubic, hexagonal, orthorhombic, etc). Mention may be made of talc, mica, silica, kaolin, polyamide (Nylon®) (Orgasol® from Atochem), poly-β-alanine and polyethylene powders, tetrafluoroethylene polymer powders (Teflon®), lauroyl-lysine, starch, boron nitride, polymeric hollow microspheres such as those of polyvinylidene chloride/acrylonitrile like Expancel® (Nobel Industrie), acrylic acid copolymers (Polytrap® from Dow Corning) and silicone resin microbeads (Tospearls® from Toshiba, for example), elastomer polyorganosiloxane particles, precipitated calcium carbonate, magnesium carbonate and hydro-carbonate, hydroxyapatite, hollow silica microspheres (Silica Beads® from Maprecos), glass or ceramic microcapsules, metallic soaps derived from carboxylic organic acids having 8 to 22 carbon atoms, preferably from 12 to 18 carbon atoms, for example zinc, magnesium or lithium stearate, zinc laurate, magnesium myristate.

As specified above, the compositions C1 and C2 are intended to be applied onto nails and/or false nails.

In particularly, they are intended to be used as photocrosslinkable nail varnish.

Preferably, the composition C1 is intended to be applied onto nails as a base coat and the composition C2 is preferably intended to be applied onto nails coated with the base coat, as a colored layer.

The radiation suitable for the crosslinking of the compositions C1 and C2 (steps b) and d) of the method according to the invention) have a wavelength between 210 nm and 600 nm, preferably between 250 nm and 420 nm, preferably between 350 nm and 410 nm. The use of lasers may also be envisaged.

In one preferred embodiment of the invention, a LED lamp or an UV lamp and particularly a mercury vapor lamp, optionally doped with further elements, such as gallium, suitable for modifying the emission spectrum of the light source, is used.

The exposure time of the deposited coat to radiation is dependent on various factors such as the chemical nature and content of the reactive compounds or the crosslinking density sought.

For nail varnishes, it would generally be sought to obtain satisfactory results for an exposure time between 10 seconds and 10 minutes, preferably between 30 seconds and 5 minutes.

Such a method may use a UV lamp having a power of approximately 36 W.

Preferably, the thickness after drying the coat of photocrosslinkable composition deposited in step a) is less than or equal to 100 µm, preferably less than or equal to 50 µm.

Preferably, the thickness of the coat of photocrosslinkable composition deposited in step c) ranges from 50 µm to 500 µm.

Following the final crosslinking step, the coat deposited on the nail or false nail may have a tacky layer on the surface thereof requiring cleaning of the crosslinked coat using for example a solvent such as isopropanol.

According to one embodiment, the method according to the invention further comprises, before steps b) and d), a period for drying the coat deposited following the respective steps a) and c), the duration whereof may vary from 10 seconds to 10 minutes, typically from 30 seconds to 5 minutes. Said drying is generally performed in air and at ambient temperature.

A particular method according to the invention solely consists of steps a), b), c) and d) as defined above, optionally at an interval of a drying period as defined above.

The crosslinked coats obtained from the crosslinking in steps b) and d) exhibit a significant stability over time, in terms of chipping resistance and gloss, particularly over the course of at least one week. They thus prove to be resistant to water, friction and shocks, and do not exhibit significant wear or chipping in this interval.

These coats are also capable of being solubilized or increasing in volume and thus weight when placed in contact with a standard makeup removal solvent. This ability to be solubilized or swell, displayed by the crosslinked coat, is specifically advantageous for the removal thereof when applied onto the surface of a nail or false nail. Indeed, the coat may be removed readily merely by means of makeup removal using a conventional solvent.

In this way, the compositions C1 and C2 are advantageously suitable for being removed using standard solvents used in the field of nail varnish, and particularly using acetone and ethyl acetate, and mixtures thereof.

According to one embodiment, the method according to the invention may comprise intermediate steps between step b) and c). Step b) may be followed by a step for applying an optionally photocrosslinkable varnish composition, particularly a colored composition, onto the coated nail obtained following step b), followed if applicable by a step for exposing the nail obtained to UV or visible light radiation.

Similarly, according to one embodiment, after the crosslinking step d), the coat deposited on the nail is coated with at least one colored composition and/or a finishing composition, also known as a *"top-coat",* these compositions being optionally photocrosslinkable.

In the way, step d) of the method according to the invention may be followed by application and UV or visible light radiation exposure steps. According to this embodiment, step d) may be followed by a step (or a plurality of steps) for applying an optionally photocrosslinkable composition, particularly a colored composition and/or a finishing composition, onto the coated nail obtained following step d), followed if applicable by a step for exposing the nail obtained to UV or visible light radiation.

Step d) may thus be followed by application / exposure cycles.

Similarly, such cycles may take place between steps b) and c).

The present invention also relates to a kit comprising:
- a photocrosslinkable cosmetic composition C1 according to the invention,
- a photocrosslinkable cosmetic composition C2 according to the invention,
- an abrasive material having a granulometry greater than or equal to 200 µm, preferably less than 300 µm, advantageously comprised from 220 µm to 280 µm, and
- a LED lamp or an UV lamp.

The present invention also relates to a makeup and/or care method of a nail and/or false nail, comprising the following steps:
i) rubbing the surface of a nail or false nail with an abrasive material having a granulometry greater than or equal to 200 µm, preferably less than 300 µm, advantageously comprised from 220 µm to 280 µm,
ii) applying a photocrosslinkable composition C1 according to the invention onto the surface of the nail or false nail which has been rubbed following step i), whereby a coat consisting of at least one layer of said photocrosslinkable composition C1 is deposited,
iii) exposing the coated nail or false nail obtained following step ii) to a LED lamp or an UV lamp, whereby photocrosslinking is carried out to obtain a crosslinked layer C'1,
iv) applying onto the nail or false nail coated with the crosslinked layer C'1, obtained following step iii), a photocrosslinkable composition C2 according to the invention, whereby a coat consisting of at least one layer of said photocrosslinkable composition C2 is deposited, and
v) exposing the coated nail or false nail obtained following step iv) to a LED lamp or an UV lamp, whereby photocrosslinking is carried out to obtain a crosslinked layer C'2.

Usually, the rubbing step is performed for less than 10 seconds, preferably less than 5 seconds, for example for approximately 3 seconds.

Throughout the application, the term "comprising a" or "including a" means "comprising at least one" or "including at least one", unless otherwise specified.

The weight percentages given in this application can be considered equivalent to the dry weight percentage of the compounds used.

The invention will be understood more clearly on reading the following description, given merely as an example.

### EXAMPLES

### Example 1

A layer of the base coat composition described below was applied onto nails.

After applying this layer, the film was crosslinked for 30 seconds under an "OPI GelColor" LED lamp from OPI.

### Base coat composition

| | |
|---|---|
| Urethane dimethacrylate (EXOTHANE 32 - ESSTECH, Inc.) | 5% |
| Nitrocellulose with 30% isopropyl alcohol (viscosity: E22 - 1/2s) | 11.63% |
| 50% acrylic copolymer in butyl acetate (PECOREZ AC 50 - PHOENIX CHEMICAL) | 21,72% |
| Ethyl acetate | 58.6496% |
| Hydroxy Cyclohexylphenyl ketone photoinitiator (Irgacure 184 - BASF) | 2% |
| Ethyl-2,4,6-trimethylbenzoylphenylphosphinate photoinitiator (Lucirin TPO-L - BASF) | 1% |
| Alizurol purple SS (Cl: 60725) (D&C VIOLET 2 - SENSIENT) | 0.0004% |

A layer of the colored composition as described hereinafter was then applied.

After applying this layer, the film was crosslinked for 30 seconds under an "OPI GelColor" LED lamp from OPI.

### Colored composition

| | |
|---|---|
| Urethane dimethacrylate (EXOTHANE 10 - ESSTECH, Inc.) | 58.79% |
| Urethane dimethacrylate (EXOTHANE 32 - ESSTECH, Inc.) | 14,69% |
| 50% acrylic copolymer in butyl acetate (PECOREZ AC 50 - PHOENIX CHEMICAL) | 2% |
| Butyl acetate | 20% |
| Titanium oxide (PI-TAO-77891 - MIYOSHI KASEI) | 0.24% |
| Red 7 lacquer (Sunchroma D&C red 7 - Sun) | 0.7% |
| Red 6 lacquer (Sunchroma D&C red 6 - Sun) | 0.58% |
| Hydroxy Cyclohexylphenyl ketone photoinitiator (Irgacure 184 - BASF) | 2% |
| Ethyl-2,4,6-trimethylbenzoylphenylphosphinate photoinitiator (Lucirin TPO-L - BASF) | 1% |

After crosslinking the final layer, the surface is cleaned with cotton wool soaked in isopropanol.

A varnish exhibiting satisfactory stability on the nail was thus obtained.

The varnish can be removed after placing in contact with acetone for 10 minutes.

### Example 2

After applying the intermediate layer according to example 1 and crosslinking same, a second layer of the colored composition according to example 1 was applied. The film was then crosslinked for 30 seconds under a "OPI GelColor" LED lamp from OPI.

One layer of the finishing composition as described hereinafter was then applied, and the film was crosslinked for 30 seconds under an "OPI GelColor" LED lamp from OPI.

After crosslinking the final layer, the surface is cleaned with cotton wool soaked in isopropanol.

### Finishing composition

| | |
|---|---|
| Urethane dimethacrylate (EXOTHANE 10 - ESSTECH, Inc.) | 58.4% |
| Urethane dimethacrylate (EXOTHANE 32 - ESSTECH, Inc.) | 14.6% |
| 50% acrylic copolymer in butyl acetate (PECOREZ AC 50 - PHOENIX CHEMICAL) | 2% |
| Butyl acetate | 19.9996% |
| Hydroxy Cyclohexylphenyl ketone photoinitiator (Irgacure 184 - BASF) | 2% |
| Ethyl-2,4,6-trimethylbenzoylphenylphosphinate photoinitiator (Lucirin TPO-L - BASF) | 3% |
| Alizurol purple SS (Cl: 60725) (D&C VIOLET 2 - SENSIENT) | 0.0004% |

A varnish exhibiting satisfactory stability on the nail was thus obtained.

The varnish can be removed after placing in contact with acetone for 10 minutes.

## Claims

1. Makeup and/or care method of a nail and/or false nail, comprising the following steps:
a) applying, onto a nail or false nail, a photocrosslinkable cosmetic composition C1, whereby a coat consisting of at least one layer of said composition C1 is deposited;
the composition C1 comprising in a physiologically acceptable medium:
- at least one photocrosslinkable (meth)acrylate compound,
- at least one film-forming polymer P1 in a proportion greater than or equal to 10% by weight in relation to the dry extract weight of the composition C1,
- at least one volatile solvent S in a proportion greater than or equal to 30% in relation to the total weight of the composition C1, and
- at least one photoinitiator,
b) exposing the coated nail or false nail obtained following step a) to UV or visible light radiation, whereby photocrosslinking is carried out to obtain a crosslinked layer C'1;
c) applying, onto the nail or false nail coated with the crosslinked layer C'1, obtained following step b), a photocrosslinkable cosmetic composition C2, whereby a coat consisting of at least one layer of said composition C2 is deposited;
the composition C2 comprising in a physiologically acceptable medium:
- at least one photocrosslinkable urethane di(meth)acrylate compound at a content by weight greater than or equal to 65% in relation to the total dry extract weight of C2, and
- at least one volatile solvent S';
d) exposing the coated nail or false nail obtained following step c) to UV or visible light radiation, whereby photocrosslinking is carried out to obtain a crosslinked layer C'2.

2. Method according to claim 1, wherein the (meth)acrylate compound is of formula (I): 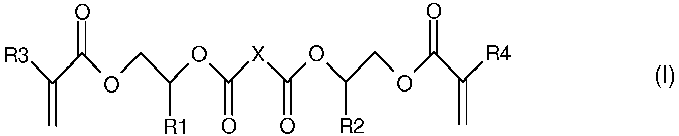 wherein:
- R1 and R2, identical or different, represent a hydrogen atom, or a methyl group, or a group of formula (II): 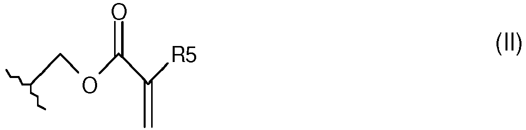 where R5 represents a hydrogen atom or a methyl group;
- R3 and R4, identical or different, represent a hydrogen atom or a methyl group;
- X represents a radical of any of the following formulae (III), (IV), (V), (VI), (VII), (VIII), (IX), or (X): 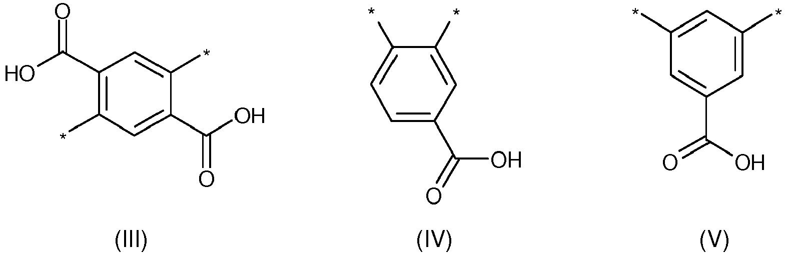 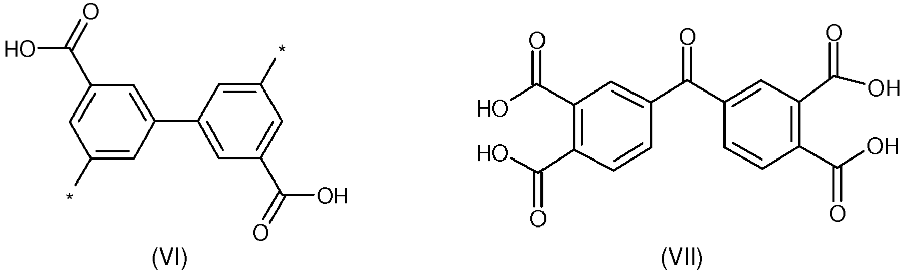 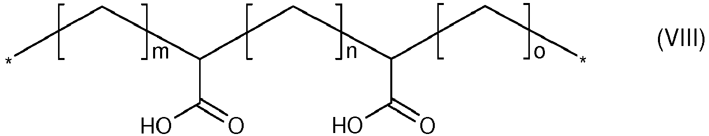 where m, n, and o, identical or different, represent an integer between 0 and 10; 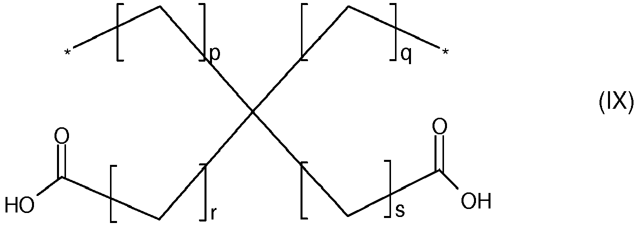 where p, q, r and s, identical or different, represent an integer between 0 and 10; 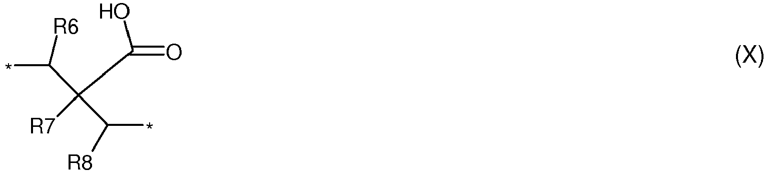 where R6, R7 and R8, identical or different, represent a hydrogen atom or a hydroxyl group.

3. Method according to claim 1, wherein the (meth)acrylate compound is a photocrosslinkable urethane (meth)acrylate compound.

4. Method according to any of claims 1 to 3, wherein the film-forming polymer P1 is chosen from the group consisting of nitrocellulose, cellulose acetopropionate, cellulose acetobutyrate, and (meth)acrylate homopolymers and copolymers, and mixtures thereof.

5. Method according to any of claims 1 to 4, wherein the composition C1 comprises at least two film-forming polymers.

6. Method according to any of claims 1 to 5, wherein the composition C1 comprises, as a film-forming polymer, a mixture of nitrocellulose and a (meth)acrylate copolymer.

7. Method according to any of claims 1 to 6, wherein the solvent S is a polar volatile solvent chosen from the group consisting of C3-C6 esters and ketones and mixtures thereof.

8. Method according to any of claims 1 to 7, wherein the solvent S is present in a content by weight ranging from 40% to 80% in relation to the total weight of the composition C1.

9. Method according to any of claims 1 to 8, wherein the photoinitiator is chosen from the group consisting of α-hydroxyketones, α-aminoketones, aromatic ketones preferably associated with a hydrogen donor compound, aromatic α-diketones, and acylphosphine oxides, and mixtures thereof.

10. Method according to any of claims 1 to 9, wherein the composition C2 comprises two photocrosslinkable urethane di(meth)acrylate compounds.

11. Method according to any of claims 1 to 10, wherein the composition C2 comprises at least 70%, preferably at least 75%, and preferentially at least 80%, by weight of photocrosslinkable urethane di(meth)acrylate compounds in relation to the total dry extract weight of C2.

12. Method according to any of claims 1 to 11, wherein the composition C2 further comprises at least one film-forming polymer P2, particularly chosen from the group consisting of nitrocellulose, cellulose acetopropionate, cellulose acetobutyrate, and (meth)acrylate homopolymers and copolymers, and mixtures thereof.

13. Method according to any of claims 1 to 12, wherein the solvent S' is a polar volatile solvent chosen from the group consisting of C3-C6 esters and ketones and mixtures thereof.

14. Method according to any of claims 1 to 13, wherein the composition C2 further comprises at least one coloring agent.

15. Method according to any of claims 1 to 14, wherein the compositions C1 and C2 comprise less than 10% by weight of (meth)acrylate monomer.

16. Method according to any of claims 1 to 15, wherein the compositions C1 and C2 comprise less than 5% by weight of (meth)acrylate monomer.

17. Kit comprising:
- a photocrosslinkable cosmetic composition C1 as defined in any one of claims 1 to 16,
- a photocrosslinkable cosmetic composition C2 as defined in any one of claims 1 to 16,
- an abrasive material having a granulometry greater than or equal to 200 µm, preferably less than 300 µm, advantageously comprised from 220 µm to 280 µm, and
- a LED lamp or an UV lamp.

18. Makeup and/or care method of a nail and/or false nail, comprising the following steps:
i) rubbing the surface of a nail or false nail with an abrasive material having a granulometry greater than or equal to 200 µm, preferably less than 300 µm, advantageously comprised from 220 µm to 280 µm,
ii) applying a photocrosslinkable composition C1 as defined in any one of claims 1 to 16 onto the surface of the nail or false nail which has been rubbed following step i), whereby a coat consisting of at least one layer of said photocrosslinkable composition C1 is deposited,
iii) exposing the coated nail or false nail obtained following step ii) to a LED lamp or an UV lamp, whereby photocrosslinking is carried out to obtain a crosslinked layer C'1,
iv) applying onto the nail or false nail coated with the crosslinked layer C'1, obtained following step iii), a photocrosslinkable composition C2 as defined in any one of claims 1 to 16, whereby a coat consisting of at least one layer of said photocrosslinkable composition C2 is deposited, and
v) exposing the coated nail or false nail obtained following step iv) to a LED lamp or an UV lamp, whereby photocrosslinking is carried out to obtain a crosslinked layer C'2.

## Patentansprüche

1. Makeup- und/oder Pflegeverfahren für einen Nagel und/oder einen falschen Nagel, die folgenden Schritte umfassend:
a) Aufbringen einer photovernetzbaren kosmetischen Zusammensetzung C1 auf einen Nagel oder einen falschen Nagel, wodurch eine Beschichtung aus mindestens einer Schicht der Zusammensetzung C1 abgeschieden wird; wobei die Zusammensetzung C1 in einem physiologisch verträglichen Medium Folgendes umfasst:
- mindestens eine photovernetzbare (Meth)acrylatverbindung,
- mindestens ein filmbildendes Polymer P1 in einem Anteil von größer oder gleich 10 Gew.-%, bezogen auf das Gewicht des Trockenextrakts der Zusammensetzung C1,
- mindestens ein flüchtiges Lösungsmittel S in einem Anteil von größer oder gleich 30%, bezogen auf das Gesamtgewicht der Zusammensetzung C1, und
- mindestens einen Photoinitiator,
b) Belichten des nach Schritt a) erhaltenen beschichteten Nagels oder falschen Nagels mit UV-Strahlung oder sichtbarer Lichtstrahlung, wodurch eine Photovernetzung durchgeführt wird, um eine vernetzte Schicht C'1 zu erhalten;
c) Aufbringen einer photovernetzbaren kosmetischen Zusammensetzung C2 auf den nach Schritt b) erhaltenen, mit der vernetzten Schicht C'1 beschichteten Nagel oder falschen Nagel, wodurch eine Beschichtung bestehend aus mindestens einer Schicht der Zusammensetzung C2 abgeschieden wird;
wobei die Zusammensetzung C2 in einem physiologisch verträglichen Medium Folgendes umfasst:
- mindestens eine photovernetzbare Urethan-di(meth)acrylatverbindung mit einem Gewichtsanteil von größer oder gleich 65% bezogen auf das Gesamtgewicht des Trockenextrakts von C2, und
- mindestens ein flüchtiges Lösungsmittel S';
d) Belichten des nach Schritt c) erhaltenen beschichteten Nagels oder falschen Nagels mit UV-Strahlung oder sichtbarer Lichtstrahlung, wodurch eine Photovernetzung durchgeführt wird, um eine vernetzte Schicht C'2 zu erhalten.

2. Verfahren nach Anspruch 1, wobei die (Meth)acrylatverbindung die Formel (I) aufweist: 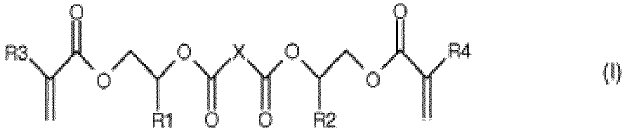 wobei:
- R1 und R2, gleich oder verschieden, für ein Wasserstoffatom oder eine Methylgruppe oder eine Gruppe der Formel (II) stehen: 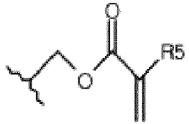 wobei R5 für ein Wasserstoffatom oder eine Methylgruppe steht;
- R3 und R4, gleich oder verschieden, für ein Wasserstoffatom oder eine Methylgruppe stehen;
- X für einen Rest einer der folgenden Formeln (III), (IV), (V), (VI), (VII), (VIII), (IX) oder (X) steht: 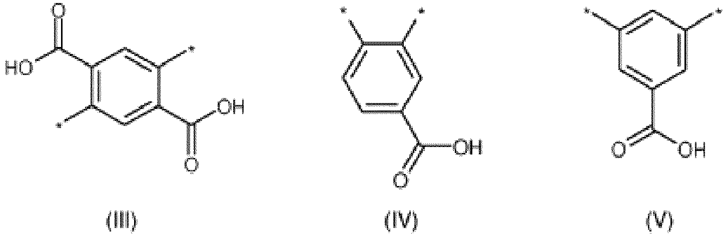 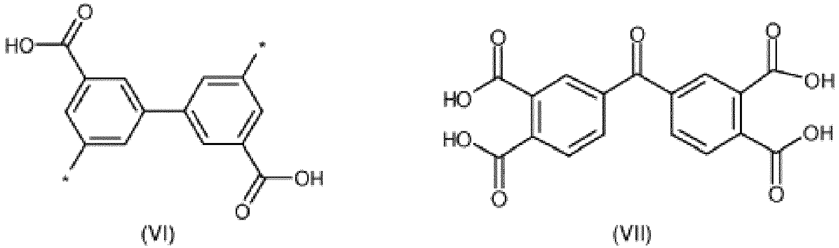 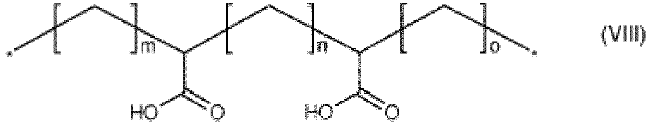 wobei m, n und o, gleich oder verschieden, für eine ganze Zahl zwischen 0 und 10 stehen; 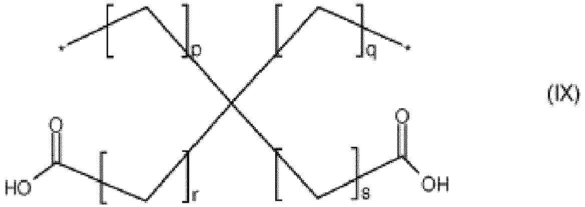 wobei p, q, r und s, gleich oder verschieden, für eine ganze Zahl zwischen 0 und 10 stehen; 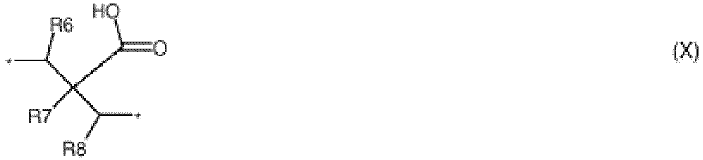 wobei R6, R7 und R8, gleich oder verschieden, für ein Wasserstoffatom oder eine Hydroxylgruppe stehen.

3. Verfahren nach Anspruch 1, wobei die (Meth)acrylatverbindung eine photovernetzbare Urethan(meth)acrylatverbindung ist.

4. Verfahren nach einem der Ansprüche 1 bis 3, wobei das filmbildende Polymer P1 ausgewählt ist aus der Gruppe bestehend aus Nitrocellulose, Celluloseacetopropionat, Celluloseacetobutyrat und (Meth)acrylathomopolymeren und -copolymeren sowie deren Mischungen.

5. Verfahren nach einem der Ansprüche 1 bis 4, wobei die Zusammensetzung C1 mindestens zwei filmbildende Polymere umfasst.

6. Verfahren nach einem der Ansprüche 1 bis 5, wobei die Zusammensetzung C1 als filmbildendes Polymer ein Gemisch aus Nitrocellulose und einem (Meth)acrylatcopolymer umfasst.

7. Verfahren nach einem der Ansprüche 1 bis 6, wobei das Lösungsmittel S ein polares flüchtiges Lösungsmittel ist, das ausgewählt ist aus der Gruppe bestehend aus C3- bis C6-Estern und -Ketonen und deren Mischungen.

8. Verfahren nach einem der Ansprüche 1 bis 7, wobei das Lösungsmittel S in einem Gewichtsanteil von 40 bis 80%, bezogen auf das Gesamtgewicht der Zusammensetzung C1, vorliegt.

9. Verfahren nach einem der Ansprüche 1 bis 8, wobei der Photoinitiator ausgewählt ist aus der Gruppe bestehend aus α-Hydroxyketonen, α-Aminoketonen, aromatischen Ketonen, die vorzugsweise mit einer Wasserstoff-Donor-Verbindung assoziiert sind, aromatischen α-Diketonen und Acylphosphinoxiden sowie deren Mischungen.

10. Verfahren nach einem der Ansprüche 1 bis 9, wobei die Zusammensetzung C2 zwei photovernetzbare Urethan-di(meth)acrylatverbindungen umfasst.

11. Verfahren nach einem der Ansprüche 1 bis 10, wobei die Zusammensetzung C2 mindestens 70 Gew.-%, vorzugsweise mindestens 75 Gew.-% und vorzugsweise mindestens 80 Gew.-% an photovernetzbaren Urethan-di(meth)acrylatverbindungen bezogen auf das Gesamtgewicht des Trockenextrakts von C2 umfasst.

12. Zusammensetzung nach einem der Ansprüche 1 bis 11, wobei die Zusammensetzung C2 ferner mindestens ein filmbildendes Polymer P2 umfasst, das insbesondere ausgewählt ist aus der Gruppe bestehend aus Nitrocellulose, Celluloseacetopropionat, Celluloseacetobutyrat und (Meth)acrylathomopolymeren und -copolymeren sowie deren Mischungen.

13. Verfahren nach einem der Ansprüche 1 bis 12, wobei das Lösungsmittel S' ein polares flüchtiges Lösungsmittel ist, das ausgewählt ist aus der Gruppe bestehend aus C3- bis C6-Estern und -Ketonen und deren Mischungen.

14. Verfahren nach einem der Ansprüche 1 bis 13, wobei die Zusammensetzung C2 ferner mindestens ein Färbemittel umfasst.

15. Verfahren nach einem der Ansprüche 1 bis 14, wobei die Zusammensetzungen C1 und C2 weniger als 10 Gew.-% (Meth)acrylatmonomer umfassen.

16. Verfahren nach einem der Ansprüche 1 bis 15, wobei die Zusammensetzungen C1 und C2 weniger als 5 Gew.-% (Meth)acrylatmonomer umfassen.

17. Kit, umfassend:
- eine photovernetzbare kosmetische Zusammensetzung C1, wie in einem der Ansprüche 1 bis 16 definiert,
- eine photovernetzbare kosmetische Zusammensetzung C2, wie in einem der Ansprüche 1 bis 16 definiert,
- ein abrasives Material mit einer Körnung von größer oder gleich 200 µm, vorzugsweise weniger als 300 µm, vorteilhafterweise zwischen 220 µm und 280 µm, und
- eine LED-Lampe oder eine UV-Lampe.

18. Makeup- und/oder Pflegeverfahren für einen Nagel und/oder einen falschen Nagel, die folgenden Schritte umfassend:
i) Reiben der Oberfläche eines Nagels oder falschen Nagels mit einem abrasiven Material mit einer Körnung von größer oder gleich 200 µm, vorzugsweise weniger als 300 µm, vorteilhafterweise zwischen 220 µm und 280 µm, und
ii) Aufbringen einer photovernetzbaren Zusammensetzung C1, wie sie in einem der Ansprüche 1 bis 16 definiert ist, auf die Oberfläche des Nagels oder des falschen Nagels, der nach Schritt i) gerieben wurde, wodurch eine Beschichtung aus mindestens einer Schicht der photovernetzbaren Zusammensetzung C1 abgeschieden wird,
iii) Belichten des nach Schritt ii) erhaltenen beschichteten Nagels oder falschen Nagels mittels einer LED-Lampe oder einer UV-Lampe, wodurch eine Photovernetzung durchgeführt wird, um eine vernetzte Schicht C'1 zu erhalten,
iv) Aufbringen einer photovernetzbaren Zusammensetzung C2, wie sie in einem der Ansprüche 1 bis 16 definiert ist, auf den nach Schritt iii) erhaltenen Nagel oder falschen Nagel, der mit der vernetzten Schicht C'1 beschichtet ist, wodurch eine Beschichtung, die aus mindestens einer Schicht der photovernetzbaren Zusammensetzung C2 besteht, abgeschieden wird, und
Belichten des nach Schritt iv) erhaltenen beschichteten Nagels oder falschen Nagels mittels einer LED-Lampe oder einer UV-Lampe, wodurch eine Photovernetzung durchgeführt wird, um eine vernetzte Schicht C'2 zu erhalten.

## Revendications

1. Procédé de maquillage et/ou de soin d'un ongle et/ou d'un faux ongle, comprenant les étapes suivantes :
a) l'application, sur un ongle ou un faux ongle, d'une composition cosmétique photoréticulable C1, de façon qu'un revêtement constitué d'au moins une couche de ladite composition C1 soit déposé ;
la composition C1 comprenant, dans un milieu physiologiquement acceptable :
- au moins un composé (méth)acrylate photoréticulable,
- au moins un polymère filmogène P1 en une proportion supérieure ou égale à 10 % en poids par rapport au poids de l'extrait sec de la composition C1,
- au moins un solvant volatil S en une proportion supérieure ou égale à 30 % par rapport au poids total de la composition C1, et
- au moins un photoamorceur,
b) l'exposition de l'ongle ou du faux ongle revêtu, obtenu suivant l'étape a), à un rayonnement de lumière visible ou UV, de façon qu'une photoréticulation soit mise en oeuvre pour que soit obtenue une couche réticulée C'1 ;
c) l'application, sur l'ongle ou le faux ongle revêtu de la couche réticulée C'1, obtenue suivant l'étape b), d'une composition cosmétique photoréticulable C2, de façon qu'un revêtement constitué d'au moins une couche de ladite composition C2 soit déposé ;
la composition C2 comprenant, dans un milieu physiologiquement acceptable :
- au moins un composé di(méth)acrylate d'uréthane photoréticulable en une teneur en poids supérieure ou égale à 65 % par rapport au poids total de l'extrait sec de C2, et
- au moins un solvant volatil S' ;
d) l'exposition de l'ongle ou du faux ongle revêtu, obtenu suivant l'étape c), à un rayonnement de lumière visible ou UV, de façon qu'une photoréticulation soit mise en oeuvre pour que soit obtenue une couche réticulée C'2.

2. Procédé selon la revendication 1, dans lequel le composé (méth)acrylate est de formule (I) : 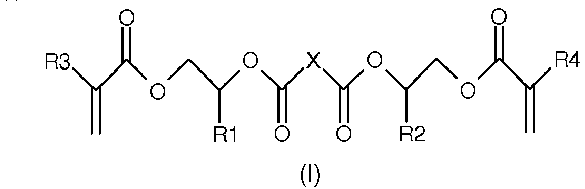 dans laquelle :
- R1 et R2, identiques ou différents, représentent un atome d'hydrogène, ou un groupe méthyle, ou un groupe de formule (II) : 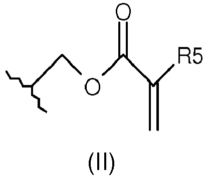 où R5 représente un atome d'hydrogène ou un groupe méthyle ;
- R3 et R4, identiques ou différents, représentent un atome d'hydrogène ou un groupe méthyle ;
- X représente un radical de l'une quelconque des formules (III), (IV), (V), (VI), (VII), (VIII), (IX) ou (X) suivantes : 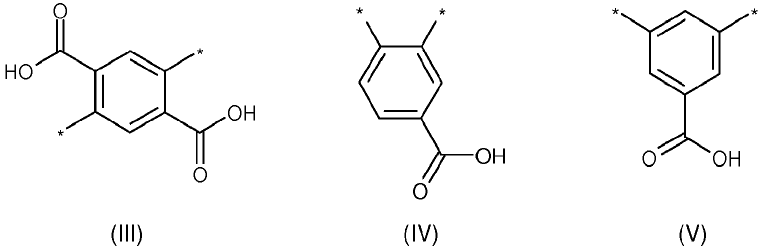 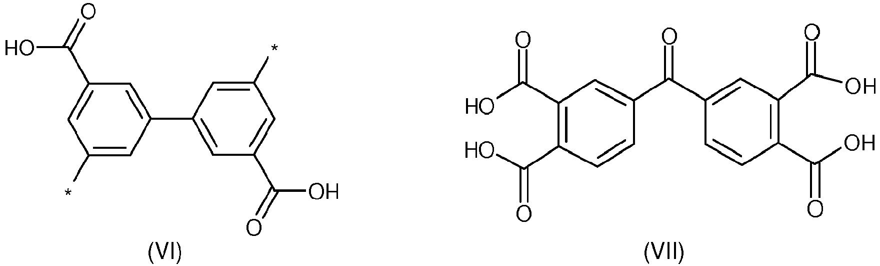 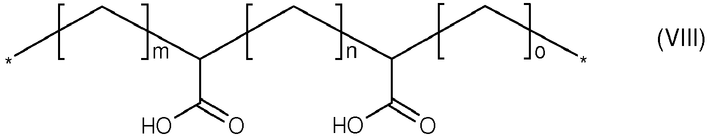 où m, n et o, identiques ou différents, représentent un entier compris entre 0 et 10 ; 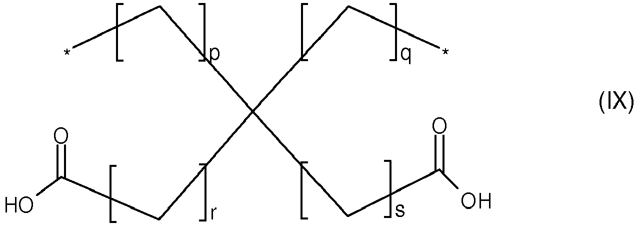 où p, q, r et s, identiques ou différents, représentent un entier compris entre 0 et 10 ; 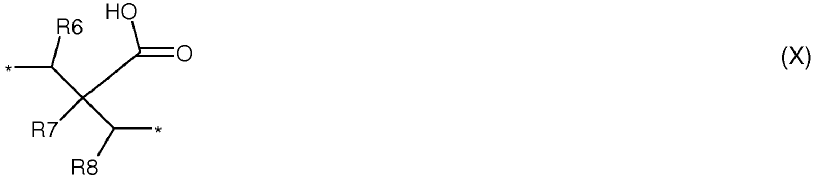 où R6, R7 et R8, identiques ou différents, représentent un atome d'hydrogène ou un groupe hydroxyle.

3. Procédé selon la revendication 1, dans lequel le composé (méth)acrylate est un composé (méth)acrylate d'uréthane photoréticulable.

4. Procédé selon l'une quelconque des revendications 1 à 3, dans lequel le polymère filmogène P1 est choisi dans l'ensemble constitué par la nitrocellulose, l'acétopropionate de cellulose, l'acétobutyrate de cellulose, et les homopolymères et copolymères de (méth)acrylate, ainsi que leurs mélanges.

5. Procédé selon l'une quelconque des revendications 1 à 4, dans lequel la composition C1 comprend au moins deux polymères filmogènes.

6. Procédé selon l'une quelconque des revendications 1 à 5, dans lequel la composition C1 comprend, en tant que polymère filmogène, un mélange de nitrocellulose et d'un copolymère de (méth)acrylate.

7. Procédé selon l'une quelconque des revendications 1 à 6, dans lequel le solvant S est un solvant volatil polaire choisi dans l'ensemble constitué par les cétones et les esters en C3 à C6, ainsi que leurs mélanges.

8. Procédé selon l'une quelconque des revendications 1 à 7, dans lequel le solvant S est présent en une teneur en poids située dans la plage allant de 40 % à 80 % par rapport au poids total de la composition C1.

9. Procédé selon l'une quelconque des revendications 1 à 8, dans lequel le photoamorceur est choisi dans le groupe constitué par les α-hydroxycétones, les α-aminocétones, les cétones aromatiques associées de préférence à un composé donneur d'hydrogène, les α-dicétones aromatiques, et les oxydes d'acylphosphine, ainsi que leurs mélanges.

10. Procédé selon l'une quelconque des revendications 1 à 9, dans lequel la composition C2 comprend deux composés di(méth)acrylates d'uréthane photoréticulables.

11. Procédé selon l'une quelconque des revendications 1 à 10, dans lequel la composition C2 comprend au moins 70 %, de préférence au moins 75 %, et mieux encore au moins 80 % en poids de composés di(méth)acrylates d'uréthane photoréticulables par rapport au poids total de l'extrait sec de C2.

12. Procédé selon l'une quelconque des revendications 1 à 11, dans lequel la composition C2 comprend en outre au moins un polymère filmogène P2, en particulier choisi dans l'ensemble constitué par la nitrocellulose, l'acétopropionate de cellulose, l'acétobutyrate de cellulose, et les homopolymères et copolymères de (méth)acrylate, ainsi que leurs mélanges.

13. Procédé selon l'une quelconque des revendications 1 à 12, dans lequel le solvant S' est un solvant volatil polaire choisi dans l'ensemble constitué par les cétones et les esters en C3 à C6, ainsi que leurs mélanges.

14. Procédé selon l'une quelconque des revendications 1 à 13, dans lequel la composition C2 comprend en outre au moins un agent colorant.

15. Procédé selon l'une quelconque des revendications 1 à 14, dans lequel les compositions C1 et C2 comprennent moins de 10 % en poids de monomère de (méth)acrylate.

16. Procédé selon l'une quelconque des revendications 1 à 15, dans lequel les compositions C1 et C2 comprennent moins de 5 % en poids de monomère de (méth)acrylate.

17. Kit comprenant :
- une composition cosmétique photoréticulable C1 telle que définie dans l'une quelconque des revendications 1 à 16,
- une composition cosmétique photoréticulable C2 telle que définie dans l'une quelconque des revendications 1 à 16,
- un matériau abrasif ayant une granulométrie supérieure ou égale à 200 µm, de préférence inférieure à 300 µm, avantageusement comprise entre 220 µm et 280 µm, et
- une lampe à LED ou une lampe à UV.

18. Procédé de maquillage et/ou de soin d'un ongle et/ou d'un faux ongle, comprenant les étapes suivantes :
i) le frottement de la surface d'un ongle ou d'un faux ongle avec un matériau abrasif ayant une granulométrie supérieure ou égale à 200 µm, de préférence inférieure à 300 µm, avantageusement comprise entre 220 µm et 280 µm,
ii) l'application d'une composition photoréticulable C1 telle que définie dans l'une quelconque des revendications 1 à 16 sur la surface de l'ongle ou du faux ongle qui a été frottée suivant l'étape i), de façon qu'un revêtement constitué d'au moins une couche de ladite composition photoréticulable C1 soit déposé,
iii) l'exposition de l'ongle ou du faux ongle revêtu, obtenu suivant l'étape ii), à une lampe à LED ou une lampe à UV, de façon qu'une photoréticulation soit mise en oeuvre pour que soit obtenue une couche réticulée C'1,
iv) l'application, sur l'ongle ou le faux ongle revêtu de la couche réticulée C'1, obtenu suivant l'étape iii), d'une composition photoréticulable C2 telle que définie dans l'une quelconque des revendications 1 à 16, de façon qu'un revêtement constitué d'au moins une couche de ladite composition photoréticulable C2 soit déposé, et
v) l'exposition de l'ongle ou du faux ongle revêtu, obtenu suivant l'étape iv), à une lampe à LED ou une lampe à UV, de façon qu'une photoréticulation soit mise en oeuvre pour que soit obtenue une couche réticulée C'2.
